# EUROPEAN PATENT APPLICATION

(11) **EP 2 949 220 A1**
(43) Date of publication of application: **02.12.2015**
(21) Application number: 14464006.7
(22) Date of filing: 28.05.2014
(51) Int. Cl.: A23K 1/18, A61K 39/36, A61K 31/192, A61K 31/35, A01N 31/08, A01N 31/16

(54) **Nutraceutic supplement for bees families and process for its administration**

(30) Priority: 27.05.2014 RO 201400392
(71) Applicant: SC Teso Spec SRL, 915200 Fundulea, Jud. Calarasi (RO)
(72) Inventor: The designation of the inventor has not yet been filed

(57) **Abstract**

The invention refer to a nutraceutical supplement for honey bees, intended to increase their tolerance to biotic and abiotic stresses. Nutraceutic supplement is made up of 00.395-0.41 parts p-coumaric acid, 0.43-0.45 parts caffeic acid, 4.15-4.3 parts maltol, 4.25-4.3 parts fructo-oligosaccharides, with less than 10 residues of hexoses per molecule, 0.97-1 parts magnesium stearate, 33.9-35 parts sodium bicarbonate, 15.5-16 parts citric acid, 11.6-12 parts tartaric acid, 23.3-24 parts of dextrose-maltose mixture, spray-crystallized, up to 100 parts water.

The administration process involves the dissolution of above composition, predosed, formulated as effervescent tablet, followed by dilution of the obtained solution with bees feed syrups, and applying of the diluted solutions, as needed, by spraying on the body of the hive, by spraying on the bee frames and honey bees, or as a stimulative nutraceutic supplement in the bees feed syrups.

## Description

This invention refers to a nutraceutical supplement for honey bees, intended to increase their tolerance to biotic and a biotic stresses, by activating the defense systems, immune / against biotic stress, produced by pathogens and parasites, and for detoxification / against abiotic stresses produced by chemical pollutants.

There are known a number of compositions for stimulation of honey bee defence systems, applied also for supplementation of feed substitutes. Substitute feed, widely used for bees foraging, based on invert sugar and/or fructose-glucose syrup, do not contain a series of compounds which are supporting the bees in their fight against parasites, pathogens and xenobiotic pollutants. Moreover, syrups based on invert sugar or isomerized glucose / blend of glucose and fructose contain hydroxymethylfurfural, a compound, which, over certain limits, has negative effects on bees (Zirbes et al., 2013, J. Agric Food Chem., 61, 11865-11870).

Honey bees are the most important pollinators used into anthropic management systems of cultivated plants. Their major economic importance is not resulted from the hive products (honey, pollen, propolis, extracts from drone larvae, etc.), but from their role in pollination. About 75% of agricultural crops depend on pollination by insects, the economic value of insect pollination being estimated at 153 billion euros (Gallai et al., Ecol. Econ. 68 2009, 810-821). Agricultural policies which have been applied in various areas of the world, such as the promotion of crops for biofuels in the European Union, significantly increase demand for pollinating honey bees (Breeze et al., 2014, PIoS One, 9, e82996). Colony Collapse Syndrome, in which the interaction between the factors related to biotic and abiotic stresses, has a major role (Staveley et al., 2014, Hum. Ecol. Risk Asses. Intl. J., 20, 566-591), causes significant economic losses and rise concerns regarding the future of the production of crops pollinated by honey bees. Sustaining the natural defense mechanisms of the honey bees against biotic stress factors, including the devastating parasite *Varroa destructor* and the viruses transmitted by this, and abiotic stress factors, and particularly the systemic insecticides, represents one of the main modalities of anthropic intervention, intended to limit these factors which are affecting honey bee populations.

Several patented compositions intended to stimulate the immune systems from bees contain natural compounds from polyphenols category. Patent US7597912 B describes the use of hop extracts, which include (phenolic) acids alpha, humulone, adhumulone, cohumulone and/or derivatives, and/or (phenolic) acids beta, lupulone, adlupulone, colupulone, and/or derivatives, for treating and preventing the attack of the parasitic mites. Patent application KR20140045726 refers to a composition for honey bees feeding which includes polyphenols from rice bran. Patent application EP 2193722 A1 claim a process for obtainment of bees feed from parts of plants rich in polyphenolic compounds.

Other compositions with are activating honey bee immune systems are those that include pre-and probiotics. Probiotic bacteria are beneficial for the health of honey bees (Vasque et al., 2012, PLoS One, 7: e33188), preventing the infection with *Paenibacillus larvae,* bacteria that produce American foulbrood (Mudronova et al., 2011, J. Api. Res., 50: 323-324), and stimulating the immune response of honey bees (Evans and Lopez, 2004, J. Econ. Entomol., 97, 752-756). Patent application US 20130064796 presents he use of the compositions which include acetic bacteria of the genus *Sacharibacter,* with one or two antagonistic bacteria which are forming spores *(Bacillus thuringiensis* and *Bacillus laterosporus),* for protection against microbial infection agents, such as *Paenibacillus larvae,* that causes American foulbrood. Patent application KR20130101370 protects a composition of honey bees feed, which includes 0.05-5 parts of probiotic bacteria of the group represented by *Lactobacillus plantarum* YML001, *Lactobacillus plantarum* YML004 and *Leuconostoc citreum* KM20.

Prebiotics, compounds which promote to the development of probiotic bacteria, have also been included in various compositions for activation of honey bee defense systems. Fructo-oligosaccharides are presented in the patent application KR 20100125738 as a prebiotic in a synbiotic combination with prebiotic bacteria from lactobacilli group, *Streptococcus faecium, Lactobacillus acidophilus, Lactobacillus lactis,* and gram positive spore forming bacilli, including *Bacillus subtilis.* Patent application RO 128464 refers to a composition for honey bee protection against pests and diseases, by activating the defense systems of bees and by limiting the development of populations of pests and pathogens, which include probiotics fructo-oligosaccharides and essential oils.

A common disadvantage of the compositions presented above is determined by the fact that these do not include components which are activating also specific detoxification genes. On honey bees the induction of the detoxification genes is accompanied by an up-regulation of genes involved in bees immunity (Mao et al., 2013, PNAS, 110: 8842-8846), reactive oxygen species being effectors and common signals for the specific metabolic pathways of both physiological responses (Nathan and Cunningham-Bussel, 2013, Nat. Rev. Immunol. 13, 349-361). Bees have a deficit of genes involved in detoxification, compared to other insects (Claudianos et al., 2006, Mol. Biol., 15, 615-636). Detoxification systems on honey bees are significantly induced only by a number of natural compounds, being much less induced by xenobiotic compounds like neonicotinoids systemic insecticides used for seed treatments or by acaricides used to limit development of the parasitic mites (Mao et al., 2013, PNAS, 110, 8842-8846). Due to this peculiarity, of common induction of both major defense systems, immune and detoxification, bees are more affected by the various xenobiotics of anthropogenic origin, comparing to other insects. Sub-lethal doses of insecticides make honey bees more susceptible to attack by pathogens (Doublet et al., 2014, Environ. Microb. doi: 10.1111/1462-2920.12426, Pettis et al. 2012, Naturwiss., 99, 153-158). A concomitant activation of enzymes involved in immunity and detoxification would trigger a synergic enhancement of honey bees tolerance to the abiotic stresses, especially xenobiotics, and to the biotic ones, produced by the attack of parasites and pathogens.

Another disadvantage of the compositions described above refers to the fact that these do not act on the mechanisms involved in honeybee social immunity, being intended for activation of defense systems at the level of individual bees. Bees, like other social insects, have evolved for the development of a social immunity, in order to combat the increased risk of transmission of diseases and parasites, resulting in specific social mode of life (Cremer et al., 2007, Curr. Biol, 17, R693-R702). Chemical messengers / pheromones are essential for the proper functioning of this social immunity (Richard and Hunt, 2013, Ins. Soc., 60, 275-291).

Food additives that act as flavor enhancers are compounds that stimulate bee social immunity because these lower the threshold for perception of volatile chemical messengers involved in this type of immunity. Maltol, widely used to enhance the flavor of foods (Leblanc and Akers, 1989, Food. Techn., 43, 18-84), was included in compositions for attracting insects (patent JPS5345368 B2) and is one of the ingredients of compositions for treating bee families. Patent RO 115411 refers to the use of maltol, formulated as effervescent tablet, for honey bees fungal diseases treatment. Patent application RO 129059 describes the use of a composition which includes maltol, dimethyl-C₁₂-C₁₆ -alkyl-benzyl-ammonium chloride, and propolis, for treatment honey bees diseases. Patent application RO 128464 associate maltol with fructo-oligosaccharides and essential oils, in order to obtain a product with a role in combating honey bees pathogens and parasites.

Although maltol is known to have an anti-oxidant action (Murakami et al. 2001, Biomed. Res., 22, 183-186), in the presence of transitional metals, especially iron, maltol can have a pro-oxidant action (Murakami et al., 2006. Biomet, 19, 253-257). A higher production of reactive oxygen species due to the maltol - iron complexes in bee tissue, interfere in the delicate balance of metabolic pathways of detoxification and immune defense, determining levels of reactive oxygen species with pathological effects. Oxidative stress resulting from such supra-physiological / pathological levels of reactive oxygen species, is involved also in olfactory memory impairment on working bees (Farooqui, 2014, Front. Gen, 5, 60). There are needed compositions which should include compounds preventing pro-oxidant action of maltolul, through which it would act antagonistic on the immune and detoxification systems from the level of individuals honey bees, and on the olfactory memory involved in social immunity, including on nestmate recognition.

Maltol inhibits lactobacteria development (Banerjee et al., 1980, Experientia, 36, 313-315) and increase bacterial cell membrane permeability (Schved et al., 1996. Lett. Appl. Microb., 22, 189-191). These characteristics are not favorable for the probiotics specific to honey bee families, fructophilic lactobacteria. These bacteria, highlighted recently (Endo et al., 2009. System. Appl. Germ., 32, 593-600), are adapted to niches high in fructose, with a reduced water activity / high osmotic pressure. The inhibitory action of maltol on lactobacteria, as well as the action of cell membrane permeability, affects the ability of fructophilic lactobacteria to survive in high osmotic pressure media. In order to maintain the maltol promoting action on pheromones communication involved in social immunity of bee families, without affecting the development of beneficial fructophilic lactobacteria, there are necessary compositions comprising ingredients which reverse the adverse effects of maltol on fructophilic lactobacteria.

Examples of such active ingredients that stimulate fructophilic lactobacteria are fructo- oligosaccharides (Dominguez et al., 2014, Food Bioproc. Technol. 7, 324-337). The fructo-oligosaccharides concentration found in honey, and which supports the development of beneficial probiotic bacteria is max. 58 mg/kg (0.058 mg/g, Mei et al., 2010, Int. J. Food Res., 17, 557-561). A higher amount of fructo-oligosaccharides, in particular of those with 3 and more fructose residues, represents an indicator of adulteration of honey with high fructose corn syrup made from inulin (Ruiz-Matute et al., 2010, J. Food Compos. Anal. 23, 273-276), thus fructo-oligosaccharides should be dosed accurately on the hive, in order to ensure the development of beneficial bees microbiocenosis, with antagonism against pathogenic micro-organisms for bees and with stimulating action of bee immunity, without generating possible problems related to suspicion of honey adulteration.

The technical problem that solves the invention is to describe a composition which includes compounds which are not antagonistic, but have complementary / synergistic actions on: (*i*) concurrent stimulation of detoxification systems and immune defense at the level of each bee; (*ii*) enhancement of pheromonal communication involved in social immunity of bee families, and (*iii*) facilitating the development of fructophilic lactobacteria beneficial for bee families. It is another object of this invention to describe a process by which this composition is administered in precise dosed amounts, according to the desired action, by spraying, on bee hive body, for further evaporation on hive, and/or on bee hive frames and on the bees, to stimulate mainly the social immunity in bees families, and/or by administration as a stimulant nutritive supplement on the bee feed, to enhance, specifically, the molecular systems involved in immune defense and detoxification, at the level of each bee.

The authors found that an equimolar combination of hydroxycinnamic acids, caffeic acid and p-coumaric acid, applied in a ratio of 1: 5 to maltol, reverse the inhibitory action of maltol of fructophilic lactobacteria. The authors also found that this mixture of hydroxycinnamic acids block the pro-oxidant effect of maltol in honey bees tissues, determining in the same time a significant stimulation of key components of molecular defense systems from bees.

Composition according to the invention is made up of 0.395-0.41 parts p-coumaric acid, 0.43-0.45 parts caffeic acid, 4.15-4.3 parts maltol, 4.25-4.3 parts fructo-oligosaccharides, with less than 10 residues of hexoses per molecule, 0.97-1 parts magnesium stearate, 33.9-35 parts sodium bicarbonate, 15.5-16 parts citric acid, 11.6-12 parts tartaric acid, 23.3-24 parts of dextrose-maltose mixture, spray-crystallized, up to 100 parts water.

The process of application of the product according to the invention comprises steps: dissolving 1 g of the above predosed composition, formulated as effervescent tablet, in 100 ml of water, followed by dilution with inverted sugar syrup or fructose-glucose syrup from corn, in a ratio of 1: 1, in the case of the solution to be used for spraying on the hive body or on the bee frames and/or on the bees, or in a ratio of 1: 9 in the case of that used as bees feed supplement, the application, at the same time, as a part of fall treatments before wintering, by spraying on the bee hive body, and by spraying on the bee frames and on the bees, of the solution formed by the dilution ratio 1:1 with bees feed, for stimulating predominantly the social immunity, or concomitant application, during the periods of beginning of foraging or during the period of lack of abundant nectar, by spraying the hive body with a solution formed by the dilution in ratio 1:1, and as stimulating nutraceutic supplement into bees feed, of the solution diluted in a ratio of 1: 9.

The invention presents the following advantages:
- Complementary and/or synergic action of the composition ingredients according to the invention on: defense systems, immune and for detoxification, at the level of each individual bees; fructophilic lactobacteria microbiocenosis from the digestive tract of individual bees and on hive level; social immunity mediated by volatile chemical messengers;
- Tableting by direct compression due to the use of spray-crystallized dextrose-maltose mixture;
- Greater stability during storage due to the use of an effervescent system in which citric acid is partially replaced by tartaric acid;
- Easy application, with rigorous compliance of the dose, even on field conditions, due to the effervescent tablet pre-dosage, which assure also solution homogenization during effervescent disaggregation;
- Not affecting commercial value of honey;
- High effectiveness in combating the fungal infection agents and adjuvant effect with natural products used for the control of parasitic mites, respectively essential oils and formic acid;
- Possibility of application as needed, by spraying on the hive body and/or bees frames and on bees, and/or by supplementing bees feed substitutes;
- Stimulation of bees feeding, due to maltolul, which is also a flavor and taste enhancer;
- Homogenize the evaporated components applied on the bottom side of inner cover, due to ventilating action done by the bees which are flying to the jar feeder installed on the inner cover.

In the followings the invention will be describe by presenting some examples of its embodiment.

*Example 1.* In a mixing vessel are mixed in geometrical proportion: 1 g magnesium stearate (Parteck^{®} Or MST, Merck, Darmstadt, Germany); 4.24 g of p-coumaric acid, IUPAC name acid (E)-3-(4-hydroxyphenyl)-2-propenoic (Sigma-Aldrich, St. Louis, Mo, USA); 4.66 g caffeic acid, IUPAC name hydrogen 3-(3,4-dihydroxyphenyl)-2-propenoic (Cayman Europe, Tallinn, Estonia); 44.5 g maltol, IUPAC name 3-Hydroxy-2-methyl-4H-piran-4-one (Otsuka Chemical, Naruto, Japan); 45.5 g fructo-oligozaride with less than 10 hexose residues (P95 Orafti^{®}, Beneo-Orafti, Tienen, Belgium); 362 g sodium bicarbonate (sodium hydrogen carbonate Emprove^{®}, Merck); 165 g citric acid, IUPAC name 2-hydroxypropane-1,2,3-tricarboxylic acid (Citric Acid Anhydrous Fine Grain 16/40, Citrique Belge, Tienen, Belgium), 124 g tartaric acid, IUPAC name 2,3-dihydroxybutanedioic acid (acid tartaric Emprove^{®}, Merck); 248 g mixture dextrose-maltose spray-crystallized (Emdex^{®}, Edward Mendell, Carmel, NY, USA). The ingredients are dried in an oven at 105 ° C for 4 hours, and then compressed directly on a tablet press EP-1 (Erweka, Heusenstamm, Germany), in molds with a diameter of 18 mm, at a pressure of 1.8-2 tons force. Tablets of 1 ± 0.035 grams, having a hardness of 9.2 to 10.5 kPa, and a disintegration time between 60 and 80 seconds are obtained. Depending on the relative humidity of the environment the composition of the tablets is: 0.395-0.41 parts p-coumaric acid, 0.43-0.45 parts caffeic acid, 4.15-4.3 parts maltol, 4.25-4.3 parts fructo-oligosaccharides, with less than 10 residues of hexoses per molecule, 0.97-1 parts magnesium stearate, 33.9-35 parts sodium bicarbonate, 15.5-16 parts citric acid, 11.6-12 parts tartaric acid, 23.3-24 parts of dextrose-maltose mixture, spray-crystallized, up to 100 parts water.

The tablets are packaged separately in tri-layer foil, polypropylene - aluminum - polyethylentereftalat, which represents a barrier to the atmospheric humidity, providing a superior stability on storage.

Optimization of the ratio of hydroxycinnamic acids, p-coumaric and caffeic, and maltol, was obtained by using experiments designed according to response surface technique, using the Software Design-Expert^{®} v. 9.0 (Stat-Ease, Minneapolis, MN, USA). Two bioassays were used, wherein the effect of different combinations of maltol and hydroxycinnamic acids, p-coumaric and caffeic, were determined, on fructophilic lactobacteria growth and on oxidative stress and on the induction of CYP9Q, enzyme from cytochrome P450 monooxigenase class, with key role in defense systems of bees (Mao et al., 2013, PNAS, 110: 8842-8846).

The strain of *Lactobacillus kunkeei* DSM 12361, one of the bees fructophilic lactobacteria beneficial to bees (Endo and Salminen, 2013, System. Appl. Microbiol., 36, 444-448) was cultivated, in presence of maltol, hydroxycinnamic acids, p-coumaric and caffeic, and mixture of maltol and hydroxycinnamic acids, p-coumaric and caffeic, in different proportions, on fructose - yeast extract - polipeptona (FYP) broth, with the following formula: 300 g/l fructose, 10 g/l yeast extract, 5 g/l polipeptone, 2 g/l sodium acetate trihydrate, 10 ml/l Tween 80, 0.02 g/l MgSO₄.7 H₂O, 0.02 g/L MnSO₄.4 H₂O, 2 g/L FeSO₄.7 H₂O, 2 g/L NaCl. The ratio 4.1 parts p-coumaric acid, 4.5 parts caffeic acid and 43 parts maltol, determined as being optimal in terms *L. kunkeei* development enhancement, was re-tested in laboratory conditions, using 400 g/l glucose-fructose syrup (Isosugar 031™, Tate and Lyle, London, United Kingdom, 74.8% dry matter, 50.2% of which is fructose), instead of 300 g/l of fructose on FYP broth. The results (tab.1) show that a hydroxycinnamic acid compensates the negative effects of maltol on the fructophilic lactobacilli.

**Tab. 1. Effect of maltolului, hidroxicinamici acid, p-coumaric, caffeic, and their mixture on development lactobacteriilor fructofilice I. kunkeei.**

| Experimental treatment | Culture development (% control) | Lactic acid production (% control) |
|---|---|---|
| FYP medium with glucose-fructose syrup (control) | 100 | 100 |
| FYP medium with glucose-fructose syrup + 21.5 mg/l maltol | 76.8 | 67,9 |
| FYP medium with glucose-fructose syrup + 2.05 mg/l p-coumaric acid, 2.25 mg/l caffeic acid | 123.5 | 112.5 |
| FYP medium with glucose-fructose syrup + 21.5 mg/l maltol, 2.05 mg/l p-coumaric acid, 2.25 mg/l caffeic acid | 107.4 | 104.8 |

To test the effect of maltol, hydroxycinnamic acids, p-coumaric and caffeic, and their mixture on the oxidative stress and induction of CYP9Q, 15 working bees 1 day old were placed in plastic boxes of 500 ml, covered with cheesecloth, and feed with 1 g of bee candy (control) or 1 g bee candy, to which have been added to the test compounds. Bee candy was made from equal parts of powdered sugar and fructose-glucose syrup (Isosugar 031™, Tate and Lyle). In fructose-syrup were homogenized in advance the tested compounds. After three days bees midguts were taken by dissection, frozen in liquid nitrogen and stored at -80 ° C, to extract total RNA for quantitative RT-PCR analysis of CYP9Q3 expression and for the determination of lipid peroxides (as malondialdehide) as markers of oxidative stress. RNA was extracted from frozen abdomens by TRIzol method (Invitrogen, Thermo Fisher Scientific, Waltham, MA, USA), treated with DNAse (Ambion^{®} protocol, Thermo Fisher Scientific) and used for the quantitative analysis of the expression CYP9Q by RT-PCR, as described by Mao et al., 2011 (PNAS, 108, 12657-12662). Lipid peroxides were determined as malondialdehid 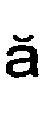 , with tiobarbituric acid (Rael et al., 2004, J. Biochem. Mol. Biol, 3, 749-752).

The results, present in table 2 below, show that hydroxycinnamic acids limit the pro-oxidant effects on honey bees tissues and determine a significant induction of CYP9Q.

**Tab. 2. Effect of maltol, hydroxycinnamic acids, p-coumaric and caffeic, and their mixture om oxidative stress and CYP9Q induction, enzyme from cytochrome P450 monooxigenazelor class, with key role in defense systems of bees.**

| Experimental treatment | Oxidative stress MDA (%) | Inducing CYP9Q (%) |
|---|---|---|
| Bee candy with sugar and fructose-glucose syrup (control) | 100% | 100% |
| Bee candy with sugar and high fructose corn syrup, glucose + 4.3 µg/g maltol | 123,7% | 102.9 |
| Bee candy with sugar and high fructose corn syrup, glucose + 0.41 µg/g p-coumaric acid and 0.45 µg/g caffeic acid | 73.5 | 164,7 |
| Bee candy with sugar and high fructose corn syrup, glucose + 4.3 µg/l maltol, 0.41 µg/l p-coumaric acid and 0.45 µg/l caffeic acid | 92.4 | 174,6 |

| | | |
|---|---|---|
| * MDA-malondialdehide, marker for oxidative stress, formed from lipid peroxides, determined with tiobarbituric acid | | |

*Example 2.* On the beginning of foraging, or during the periods of lack of abundant foraging, it is applied a first type of combined treatment. An effervescent tablet of 1 g is dissolved in 100 ml of drinking water, followed by dilution with inverted sugar syrup or glucose-fructose syrup from corn, in the ratio of 1: 1, in the case of the solution to be used for spraying the hive body, or a ratio of 1: 9 of that used as a supplement in feeding bees. Solution, diluted in ratio of 1 : 1 with bees feed is applied by spraying on bottom side of the inner cover of the bee frames, for stimulating mainly the social immunity, mediated by volatile chemical compounds. The solution diluted in a ratio of 1: 9 applied on the feeding jar from the upper side of the inner cover. Bee attraction, in the area of evaporation of the solution applied on the bottom side of the inner cover, promote the exposure of the bees, and of the parasites from the bees, to the action of maltol vapors and homogenize the vapors into the hive, under the action of air currents related to the flight of bee into the hive.

The application process was tested in the spring of 2014, in a period with temporary lack of abundant nectar, between the ends of foraging on black locust and before the beginning of foraging on sunflower. The experiment was done in only one apiary, and included the following experimental treatments: V₁ -untreated control; V₂ -treatment with 1.8 kilograms of invert sugar syrup (Apiinvert^{®}, Agrana, Vienna, Austria) in which were homogenized 200 ml of the solution, resulted from dissolution of 2 effervescent tablets of 1 g, acc. Ex. 1, applied on the feeding jar; V₃ - treatment with 200 g of solution, resulting by diluting 100 ml solution, wherein was dissolved 1 g effervescent tablet acc. Ex. 1, with 100 g inverted sugar syrup (Apiinvert^{®}, Agrana), by spraying on the inner cover; V₄ -combined treatment, treatment with 200 g of solution, resulting by diluting 100 ml solution, wherein was dissolved 1 g effervescent tablet acc. Ex. 1, with 100 g inverted sugar syrup (Apiinvert®, Agrana), by spraying on the inner cover treatment with 1.8 kilograms of invert sugar syrup (Apiinvert®, Agrana) in which were homogenized 200 ml of the solution, resulted from dissolution of 2 effervescent tablets of 1 g, acc. Ex. 1, applied on the feeding jar. Each experimental treatment included 4 repetitions, each repetition consisting of 3 bee families, each bee family having 15,000 bees; randomization of the 4 treatments in 4 repetitions was done in a Latin square schedule.

The hives were weighed at the beginning of the period of experimentation and after 4 weeks of experimentation. Monitoring of the effects on *Varroa* during the experimental period was done by using a cardboard sheet placed on the anti*-Varroa* bottom board, on which were registered the death mites. Monitoring of the evolution of ascospherosis was done by daily numbering of the death larvae (chalkbrood, symptom of infection with *Ascosphaera apis),* removed at the beehive entrance. The data were processed by analysis of variance (10 Stats, StatSoft, Tulsa, OK, USA).

The results are presented in table 3. These results demonstrate a good efficacy of the product done and applied according to invention, concomitantly, by spraying of the solution acting by evaporation and as supplement in the bee feed, of the solution which stimulate the defense system at the level of each bee.

**Tab. 3. The efficacy of the product in accordance with the invention, applied concomitantly as spraying of the solution which acts by evaporation and as a supplement in the bees feed of the solution for bee stimulation.**

| Experimental treatment | Death mites (average number per week) | Chalkbrood (average number per week) | Hive mass variation (+ Kg) |
|---|---|---|---|
| V₁ untreated control | 172c | 224c | 2.25 c |
| V₂ treatment with 1.8 kilograms of invert sugar syrup (Apiinvert®, Agrana, Vienna, Austria) in which was homogenized 200 ml of the solution resulted from dissolution of 2 effervescent tablets of 1 g, acc. Ex. 1, applied on the feeding jar | 368ab | 98b | 3.07b |
| V₃ treatment with 200 g of solution, resulting by diluting 100 ml solution, wherein was dissolved 1 g effervescent tablet acc. Ex. 1, with 100 g inverted sugar syrup (Apiinvert®, Agrana), by spraying on inner cover | 312b | 105b | 2.94b |
| V₄ - combined treatment, treatment with 200 g of solution, resulting by diluting 100 ml solution, wherein was dissolved 1 g effervescent tablet acc. Ex. 1, with 100 g inverted sugar syrup (Apiinvert®, Agrana), by spraying on the inner cover treatment with 1.8 kilograms of invert sugar syrup (Apiinvert®, Agrana) in which were homogenized 200 ml of the solution, resulted from dissolution of 2 effervescent tablets of 1 g, acc. Ex. 1, applied on the feeding jar | 402a | 72a | 3.36a |

| | | | |
|---|---|---|---|
| * values followed by the same letter do not differ significantly for P > 0.05 | | | |

*Example 3.* During the treatments on the bee families during the fall, as preparation for wintering, it is applied a second type of combined treatment. An effervescent tablet of 1 g is dissolved in 100 ml of drinking water, followed by dilution with inverted sugar syrup or fructose-glucose syrup from corn, in the ratio of 1: 1. One portion of the resulting solution is applied by spraying on the lower part of inner-cover from the top of the bee frames, for prevalent stimulation of the social immunity mediated by volatile chemical compounds, and by spraying at the same time, on the bee frames and on bees, with an another portion of solution, for stimulation of the mutual cleaning behavior of infested bee. It is recommended that this combined treatment to be done together with the application of a treatment with volatile anti-*Varroa* products, natural essential oils or formic acid. The purpose of such treatment is to achieve a combination of hive protection actions against the attack of *Varroa destructor,* by combining the -cidal effect of the vapors, with the social immune effect of disinfestation by mutual cleaning of infested bee ("grooming"), stimulated by spraying with syrup which represent bee feeding, mixed with maltol, hydroxycinnamic acids, p-coumaric and caffeic, and fructo-oligosaccharides

The application process was experimented in the autumn of year 2013, at the end of September, in a period when the average temperature during the day varied between 16.8 and 23.5°C, being over the threshold of 15°C recommended for treatments on which thymol is applied for subsequent evaporation. The experiment was done in only one apiary and included the following experimental treatments: V₁ - untreated control; V₂ - treatment with a standard product based on thymol applied by evaporation (Apiguard^{®}, 2 trays with 50 g gels, 12.5 g thymol per tray, above the frames, under inner cover); V₃ - treatment with a standard product based on thymol applied by evaporation (Apiguard^{®}, 2 trays with 50 g gels, 12.5 g thymol per tray, above the frames, under inner cover), and with 200 g of solution, resulting after dilution of 100 ml of the solution wherein was dissolved 1 effervescent tablet of 1 g done acc. Ex. 1, with 100 g fructose-glucose syrup from corn (Isosugar 031™, Tate and Lyle) applied by spraying inner cover; V₄ -treatment with a standard product based thymol applied evaporation (Apiguard^{®}, 2 trays above the frames, under inner cover), and with 200 g of solution, resulting after dilution of 100 ml of the solution wherein was dissolved 1 effervescent tablet of 1 g done acc. Ex. 1, with 100 g fructose-glucose syrup from corn (Isosugar 031TM, Tate and Lyle), applied by spraying of the bee frames and of bee with a hand held sprayer, on the bee frames exposed by partial raising of the upper body(ies) / shallow super; V₅ -treatment with 200 g of solution, resulting after dilution of 100 ml of the solution wherein was dissolved 1 effervescent tablet of 1 g done acc. Ex. 1, with 100 g fructose-glucose syrup from corn (Isosugar 031™, Tate and Lyle), applied by spraying on the inner cover and on the bee and bee frames. Each treatment included four repetitions, each repetition consisting of 3 bee families, each bee family having 15,000 bees; randomization of the 4 treatments in 4 repetitions was done in a Latin square.

Before the application of the treatments were taken 300 bees from three bee frames, on each was determined the degree of infestation with Varroa by using the method proposed by De Jong et al., 1982, Apidologie 13:297-303. For diagnostic of the presence of *Varroa* on brood were inspected, by opening, 100 combs with workers brood from each bee family. The bee families were separated on three category of infestation with *Varroa,* low, medium and high, based on the results from the infestation determination, and each repetition included one bee family from each category of infestation. The monitoring of the effect of products during the experimental period was done by using a cardboard sheet placed on the anti-*Varroa* bottom board, on which were registered the death mites. On the end of the experimentation period was done once again the estimation of the degree of infestation on the bee hives used in experiment, by taken-out 300 bees per bee family and 100 larvae. Efficacy of each treatment was estimated by calculation of an index of reduction = (initial index - final index) / initial index x 100. The data obtained during the experiment were processed by analysis of variance (Statistica 10, StatSoft, Tulsa, OK, US). The results are presented in table 4.

**Tab. 4. The efficacy of the product in accordance with the invention, spraying performed at the same time, on the hive body of solution acting by evaporation, and on bees frames and bees, of the bees stimulating solution***

| Treatment | Anti-Varroa efficacy (%) | Death mites (average number per week) | Chalkbrood (average number per week) |
|---|---|---|---|
| V₁ untreated control | - | 187e | 164d |
| V₂ Apiguard ^{®}, 2 trays of 50 g 12.5 g Thymol gel per tray | 63, 2b | 537c | 132c |
| V₃ Apiguard ^{®}, 2 trays of gel 50 g; 200 g solution obtained by diluting 100 ml solution wherein was dissolved 1 g tablet Ex. 1, with 100 g fructose-glucose syrup, inner cover sprayed | 67, 4ab | 642b | 98b |
| V₄ -Apiguard ®, 2 trays of gel 50 g; 200 g obtained by diluting 100 ml solution wherein was dissolved 1 g tablet Ex. 1, with 100 g fructose-glucose syrup, applied by spraying the bees and frames | 72, 5a | 797a | 105b |
| V₅ -treatment with 200 obtained by diluting 100 ml solution wherein was dissolved 1 g tablet Ex. 1, with 100 g fructose-glucose syrup, applied by spraying on the inner cover and on the frames of bees and bees. | 51, 2 c | 424d | 72a |

| | | | |
|---|---|---|---|
| * values are followed by the same letter does not differ significantly for P > 0.05 | | | |

The concomitant application of commercial gel product working by evaporation and by spraying bees frames and bees with the solution containing 1 g/l composition according to Ex. 1, diluted in fructose-glucose syrup in the ratio of 1:1, resulted in a significant increase in efficacy against *Varroa destructor* mites. An increase in the control efficacy is resulted also when a solution of 1 g/l of composition in accordance Ex. 1, diluted in glucose-fructose syrup, in the ratio of 1: 1, is sprayed on the inner cover. This is higher than that of the commercial compound gel applied alone, but at the limit of statistical assurance.

The solution of 1 g/l of composition in accordance with Ex. 1, diluted in glucose-fructose syrup, 1:1, applied as combined treatment, by spraying the inner cover and on bees frames and on bees, has an parasitic mite controlling efficacy under the commercial product containing thymol, but the efficacy of fungal disease control is significantly increased.

These results demonstrate the efficacy of the product obtained according to the invention, applied in accordance with the invention, by spraying on the same time, on the hive body of the solution working by evaporation, and on the bees frames and bees, of the solution which stimulate the mutual cleaning behavior of bees. The composition includes pollen characteristic components, p-coumaric acid, present in particular pollen, sporopolenine and oleoresins from propolis - caffeic acid. This composition with the characteristic ingredients of pollen and propolis has a high effectiveness in combating the fungal infection agent and has also an adjuvant effect with the natural products used for parasitic mites control. Boosting defense systems, at the level of individual bees and on hive level, determined also a stimulation of the development of bee families during periods of temporary lack of abundant nectar.

## Claims

1. Composition according to the invention **characterized in that** is made up of 0.395-0.41 parts p-coumaric acid, 0.43-0.45 parts caffeic acid, 4.15-4.3 parts maltol, 4.25-4.3 parts fructo-oligosaccharides, with less than 10 residues of hexoses per molecule, 0.97-1 parts magnesium stearate, 33.9-35 parts sodium bicarbonate, 15.5-16 parts citric acid, 11.6-12 parts tartaric acid, 23.3-24 parts of dextrose-maltose mixture, spray-crystallized, up to 100 parts water.

2. The process of application of the composition according to the invention **characterized in that** comprises the following steps: dissolving 1 g of the above predosed composition, formulated as effervescent tablet, in 100 ml of water, followed by dilution with inverted sugar syrup or fructose-glucose syrup from corn, in a ratio of 1: 1, in the case of the solution to be used for spraying on the hive body or on the bee frames and/or on the bees, or in a ratio of 1: 9 in the case of that used as bees feed supplement, the application, at the same time, as a part of fall treatments before wintering, by spraying on the bee hive body, and by spraying on the bee frame and the bees, of the solution formed by the dilution ratio 1:1 with bees feed, for stimulating predominantly the social immunity, or concomitant application, during the periods of beginning of foraging or during the period of lack of abundant nectar, by spraying the hive body with a solution formed by the dilution in ratio 1:1, and as stimulating nutraceutic supplement into bees feed, of the solution diluted in a ratio of 1: 9.
